(19) **Europäisches Patentamt**

**European Patent Office**

**Office européen des brevets**

(11) Publication number : **0 460 827 B1**

# EUROPEAN PATENT SPECIFICATION

(45) Date of publication of patent specification :
27.07.94 Bulletin 94/30

(51) Int. Cl.⁵ : **C07C 15/44,** C07C 5/333,
B01J 23/94

(21) Application number : 91304600.9

(22) Date of filing : 21.05.91

(54) Production of alpha, para-dimethylstyrene.

(30) Priority : 08.06.90 GB 9012812

(43) Date of publication of application :
11.12.91 Bulletin 91/50

(45) Publication of the grant of the patent :
27.07.94 Bulletin 94/30

(84) Designated Contracting States :
AT BE CH DE DK ES FR GB GR IT LI NL SE

(56) References cited :
US-A- 4 590 324
CHEMICAL ABSTRACTS, vol. 73, 31st August
1970, page 247, abstract no. 44616s,Columbus,
Ohio, US; J. BLANCA ALVAREZ: "Catalytic
dehydrogenation of p-cymene", & REV. REAL
ACAD. CIENC. EXACTAS, FIS. NATUR. MAD-
RID 1969, 63(4), 811-901
CHEMICAL ABSTRACTS, vol. 68, 1st January
1968, page 243, abstract no. 2648v,Columbus,
Ohio, US; G. KREMENIC et al.: "Preparation of
alpha,p-dimethylstyrene by catalytic dehyd-
rogenation of p-cymene on ZnO-Cr2O3", & AN.
REAL SOC. ESPAN. FIS. QUIM., SER. B 63(7-8),
795-807 (1967)

(73) Proprietor : **BP Chemicals Limited**
**Britannic House**
**1 Finsbury Circus**
**London EC2M 7BA (GB)**

(72) Inventor : **Lawrenson, Malcolm John**
**BP Chemicals Limited,**
**Salt End**
**Hedon, Hull HU12 8DS (GB)**
Inventor : **Smith, Paul Christopher J., BP**
**Chemicals Limited**
**Belgrave House,**
**76 Buckingham Palace Road**
**London SW1W 0SU (GB)**

(74) Representative : **Krishnan, Suryanarayana**
**Kalyana et al**
**BP INTERNATIONAL LIMITED**
**Patents & Agreements Division**
**Chertsey Road**
**Sunbury-on-Thames Middlesex TW16 7LN**
**(GB)**

## Description

The present invention relates to a method of producing alpha, para-dimethylstyrene (hereafter "APDMS") by a catalytic dehydrogenation of para-cymene.

APDMS is a valuable intermediate for the manufacture of perfumes with musk-like fragrance such as 1,1,3,4,4,6-hexamethyl 1,2,3,4-tetrahydronaphthalene (hereafter referred to as "HMT") and its 7-acetyl derivative (hereafter referred to as "Acetyl HMT"). These products are sold under the trade names "TONALID", "GALOXOLIDE" and "VERITONE" (all Regd Trade Marks). Such products and their preparation are described in GB-A-1442954 and in US-A-3246044. However, in the former patent specification it is stated at page 1, lines 26-31 that:

"US Patent No. 3246044 discloses a simple convenient process for the production of HMT and Acetyl HMT utilizing as reactants alpha, para-dimethylstyrene and neo-hexene (t-butylethylene). However, the alpha, para-dimethylstyrene required in the process of US Patent 3246044, previously in good supply as a by-product of p-cresol ex p-cymene process, is now only available in limited supply and is likely to become even scarcer in the future."

GB-A-1442954 was filed in 1973 and even today, nearly 20 years after the filing date of this application, there is a scarcity of alpha, para-dimethylstyrene. Thus there is at present no commercially viable synthetic route to APDMS.

It is well known to dehydropenate ethyl benzene to styrene using a dehydrogenation catalyst. Such processes have been claimed and described inter alia in our published EP-A-336622, and in GB-A-1176916, GB-A-1378151, US-A-3654181, US-A-3702346, US-A-3703593, US-A-4048244, US-A-4048245, and US-A-4652687. Whilst all these prior publications include a long list of hydrocarbons, both aliphatic and aromatic, which can be dehydrogenated to produce the corresponding olefinic material from saturated hydrocarbons, none of them include para-cymene as a hydrocarbon which can be dehydrogenated by the methods claimed in these prior patents.

A further list of patents mention a dehydrogenation reaction which is followed by an oxidation step and in these patents p-isopropyl toluene (which is the same as para-cymene) is inter alia listed although no specific examples of dehydrogenation of para-cymene to APDMS is described. These patents include: US Patents 4418237, 4435607, 4565898, 4652687, 4691071 and 4717779.

The use of an oxidation step and the presence of oxygen as a reactant not only make the process capital intensive thereby adversely affecting the economics of the process but can also run the risk of forming explosive mixtures with the hydrocarbons and can in some instances deactivate the catalyst.

It has now been found that the scarcity of APDMS can be met by the dehydrogenation of p-cymene using a process which avoids the use of any oxygen during the reaction.

Accordingly, the present invention is a process for the production of alpha, para-dimethylstyrene, said process comprising dehydrogenating para-cyemene in the substantial absence of oxygen or an oxidising gas and in the presence of an iron oxide, $Fe_3O_4$, catalyst and steam at a temperature in the range from 500-700°C and a pressure from 0.1-10 atmospheres.

By the expression "in the substantial absence of oxygen or an oxidising gas" as used herein and throughout the specification is meant that to carry out the dehydrogenation of para-cymene to APDMS at no stage is any oxygen or oxidising gas introduced into the system and any such gases present in the reaction system prior to the commencement of the reaction is purged from the dehydrogenation reactor. For the purposes of this invention, the co-reactant steam is not regarded as an oxidising gas. Ideally the dehydrogenation reaction should be totally free of any oxygen or oxidising gases as herein defined.

The catalyst used for the dehydrogenation is the iron oxide catalyst, $Fe_3O_4$. This catalyst species can be derived by the reduction of a commercially available $Fe_2O_3$ catalyst. The reduction of $Fe_2O_3$ can be carried out prior to use in the dehydrogenation step or 'in situ', for instance by conditioning over a duration in a dehydrogenation reactor where significant quantities of hydrogen will be readily available for this purpose. Examples of commercial iron oxide catalysts that may be used include Girdler G64C (ex Sud-Chemie) and Shell 105. The performance of these catalysts may be improved by the use of promoters such as potassium.

Prior to the introduction of the catalyst into the reactor it is essential to ensure that the reactor is substantially free of any oxygen or other oxidising gases. This is due to the fact that the presence of such gases may risk deactivating the iron oxide catalyst. The removal of oxygen or other oxidising gases in the reactor may be achieved by purging the reactor with a gas inert under the reaction conditions such as e.g. nitrogen.

In carrying out the dehydrogenation reaction, the relative molar ratios of steam to para-cymene in the feed is suitably from 1.5 : 1 to 20 : 1, preferably from 5 : 1 to 10 : 1.

The dehydrogenation reaction is carried out at a temperature from 500-700°C, preferably from 520-600°C. The reaction pressure is preferably from 0.1-1 atmosphere.

The process of the present invention can be carried out isothermally or adiabatically.

The dehydrogenation reaction is suitably carried out using a liquid hourly space velocity (LHSV) in the range of 0.1-10hr⁻¹, preferably from 0.1-2hr⁻¹ based on the para-cymene feed.

It is expected that the iron oxide catalyst will be deactivated during the dehydrogenation reaction due to coking. In the case of the present invention, it is not possible to regenerate the catalyst by the conventional auto-regeneration methods. The coked catalyst has to be regenerated by steam in the absence of any hydrocarbon such as the feed hydrocarbon. The deactivated catalyst used in this reaction can however be regenerated without removing the same from the reactor by shutting off the hydrocarbon feed to the reactor and simply heating the deactivated catalyst in the presence of steam alone. Once the regeneration of the catalyst is achieved, the supply of the hydrocarbon to the reactor can be resumed and the dehydrogenation continued as previously. Of course, it would be possible also to remove the catalyst from the reactor and to introduce a fresh charge of the catalyst. However, this would necessitate shutdown of the process, and therefore, it is more preferable to regenerate the catalyst in situ by steaming.

By operating the process as described above it is possible to readily achieve conversions in the region of 60-80% and selectivities of the order of 95-99%. In order to improve the conversion and selectivity of the reaction it may sometimes be an advantage to carry out the reaction in more than one reactor so that the effluent from the first reactor is fed into a second reactor containing a further aliquot/charge of a fresh catalyst. Such expedients will be well known to those skilled in the art.

The alpha, para-dimethylstyrene so formed can be recovered from the reaction products by fractional distillation. Small amounts of by-products which can be formed by this process include methyl styrenes and non-aromatic cyclohexenes in amounts from 1-5%. Methyl styrenes form the light ends boiling from 160-170°C, the feed hydrocarbon boils at 176°C whereas APDMS boils at ca 185°C.

Whilst methyl styrenes have to be removed before the APDMS product is used in synthesis of perfumery products such as Tonalid, the non-aromatics need not be separated for the subsequent reaction.

The present invention is further illustrated with reference to the following Examples:

EXAMPLES

A. General Procedure - Conditioning of Catalyst

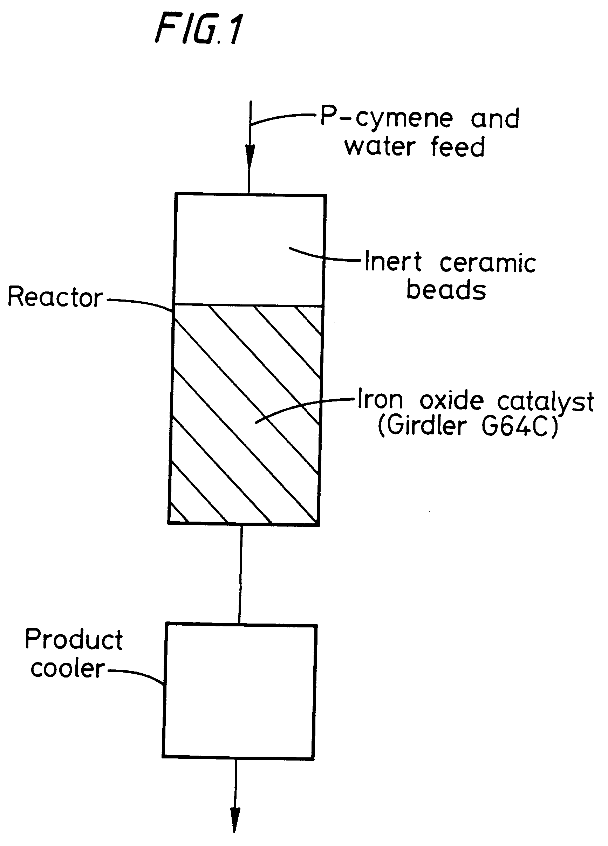

Fig. 1 shows the experimental set-up used for the Examples described below. A reactor (18 in. long, 1 in. I.D.) was charged with 100 g (80ml) of a commercial iron oxide catalyst (Girdler G64C, ex Sud-Chemie). The remaining space at the top of the reactor was filled with inert diluent in the form of cylindrical ceramic beads (4mm x 4mm), which acted as a preheating zone.

This iron oxide catalyst was pre-conditioned for two days using ethylbenzene (hereafter "EB") feed at 600°C, atmospheric pressure, LHSV 0.56hr⁻¹ and a steam/ethylbenzene ratio of 1.75w/w. This pre-conditioning period reduced the iron oxide ($Fe_2O_3$) catalyst in situ to the more active haemetite form ($Fe_3O_4$). It is also possible to carry out this pre-conditioning procedure using p-cymene feed rather than EB.

Thereafter, the temperature was reduced to 560°C and allowed to stabilise for two hours. The EB feed was then replaced by p-cymene feed of 98.2% purity. The feed rate and steam ratio were also altered when p-cymene was introduced into the reactor. The p-cymene feed rate was reduced to a space velocity of 0.30h⁻¹ and the steam/p-cymene ratio was increased to 6 w/w.

In the following Examples, the product analyses were all carried out by off-line Gas Chromatography using the following process and apparatus:

Gas Chromatopraphy Analysis Method

| Equipment (GC) | : Pye Unicam PU4500 |
| Column Used | : SP1000 |
| Stationary Phase | : Modified Polyethylene Glycol |
| Carrier Gas | : Helium |
| Helium Flow rate | : 1ml/min |
| Helium Pressure | : 9-10 psi |
| Sample Injected | : 0.2 microlitres |
| Splitter Ratio | : 1:150 |
| Initial Column Temperature | : 80°C; hold for 8 mins |
| Rate of Temperature Increase | : 4°C/min |
| Final Column Temperature | : 150°C |
| (Achieved after 25.5 mins) | |

Retention Times

| p-Cymene (feed) | : 13-14 mins |
| alpha, p-Dimethylstyrene (product) | : 19-20 mins |

The by-products were calculated from a GC mass spectrum of the product samples. The results from the Examples are summarised in Table 1 below:

Example 1

The p-cymene feed to the reactor containing the conditioned haemetite catalyst produced as described in (A) above was continued for 10hr, after which the product from the reactor was sampled by GC. The results showed a good conversion of 58.6% with a high selectivity of 97.5 mole % to teh desired product.

Example 2

The procedure of Example 1 was continued for 18 hr (total 28 Hours-on-Stream (HOS)) the p-cymene feed was then stopped, whilst maintaining the steam flow in order to regenerate the catalyst by steaming for about 1 hr at a slightly lower temperature of about 550°C to remove any coke deposits from the catalyst surface.

After regeneration, the reactor temperature was increased to 580°C, the p-cymene feed to the reactor restored at the same steam ratio and space velocity as in Example 1. After two hours (total HOS 30 hr) the reactor product was sampled and analysed by GC. The conversion achieved (69.1%) was higher than that at 560°C and the selectivity was still high (95.0 mol%).

Example 3

The reaction in Example 2 was continued for a further 4 hours (total 34 HOS) under the same conditions, after which a product sample was again analysed by GC. The conversion was found to be 54.7% and the selectivity was high at 95.4 mol%.

Example 4

The reaction in Example 3 was continued for a further 6 hours (total 40 HOS) and the catalyst was then regenerated by steaming for 1hr at 550°C as in Example 2. Thereafter, p-cymene feed was re-introduced, with a reactor temperature of 550°C, the other reaction conditions remaining unchanged. After a 2hr run (total 42 HOS) a sample of the product was analysed by GC. The conversion was found to be 63.8% and the selectivity 97.9 mol%.

Example 5

The reaction in Example 4 was continued for a further 44 hr (total 86 HOS) under the same conditions and a sample of the reactor product analysed again by GC. The conversion was found to be 40.9% and selectivity 97.8 mol%. This result showed that catalyst ageing was much slower at lower temperatures than at the relatively higher temperature of 580°C used in Example 3.

Example 6

The catalyst after 86 HOS in Example 5 was steamed again for 2 hr as in Example 2. The p-cymene feed was then re-introduced and the reaction continued under the same conditions as in Example 5 for 2 hr (total 88 HOS). A product sample from the reactor was then analysed by GC and was found to show a conversion of 67.3% and a selectivity of 98.1 mol%. When compared with the results in Example 4, this result shows that the activity of the catalyst can be completely restored by the regeneration procedure used.

TABLE 1 DEHYDROGENATION OF P-CYMENE USING GIRDLER G64C CATALYST

| Example No. | Hours on Stream | Temp Deg C | s/o Ratio wt/wt | LHSV $h^{-1}$ | Conversion % | Selectivity mol % |
|---|---|---|---|---|---|---|
| 1 | 10 | 560 | 6:1 | 0.30 | 58.6 | 97.5 |
| 2 | 30 | 580 | 6:1 | 0.30 | 69.1 | 95.0 |
| 3 | 34 | 580 | 6:1 | 0.30 | 54.7 | 95.4 |
| 4 | 42 | 550 | 6:1 | 0.30 | 63.8 | 97.9 |
| 5 | 86 | 550 | 6:1 | 0.30 | 40.9 | 97.8 |
| 6 | 88 | 550 | 6:1 | 0.30 | 67.3 | 98.1 |

**Claims**

1. A process for the production of alpha, para-dimethyl styrene, said process comprising dehydrogenating para-cymene in a reactor in the substantial absence of oxygen or an oxidising gas and in the presence of an iron oxide, $Fe_3O_4$, catalyst and steam at a temperature in the range from 500-700°C and a pressure from 0.1-10 atmospheres.

2. A process according to Claim 1 wherein the iron oxide catalyst is derived by the reduction of a commercially available $Fe_2O_3$ catalyst.

3. A process according to Claim 2 wherein the reduction of $Fe_2O_3$ is carried out in situ prior to the dehydrogenation step.

4. A process according to any one of the preceding Claims wherein the reactor is rendered substantially free of oxygen or any oxidising gases prior to introduction of the dehydrogenation catalyst into the reactor.

5. A process according to any one of the preceding claims wherein the dehyrogenation reaction is carried out using a feed in which the relative molar ratio of steam to para-cymene is from 1.5 : 1 to 20 : 1.

6. A process according to any one of the preceding Claims wherein the dehydrogenation is carried out at a temperature from 520-600°C.

7. A process according to any one of the preceding Claims wherein the dehydrogenation is carried out using a liquid hourly space velocity (LHSV) in the range of 0.1-1-10 hr⁻¹ based on the para-cymene in the feed.

**8.** A process according to any one of the preceding Claims wherein any deactivated catalyst in the reactor is regenerated by heating the deactivated catalyst in the presence of steam.

**9.** A process according to Claim 8 wherein the deactivated catalyst is regenerated in the dehyrogenation reactor in situ by shutting off the hydrocarbon feed to the reactor and heating the deactivated catalyst in the presence of steam.

**10.** A process according to any of the preceding Claims wherein the alpha, para-dimethyl styrene formed is recovered from the reaction products by fractional distillation.


**Patentansprüche**

**1.** Verfahren für die Herstellung von alpha,para-Dimethylstyrol, wobei besagtes Verfahren die Dehydrierung von para-Cymol in einem Reaktor in praktischer Abwesenheit von Sauerstoff oder einem oxidierenden Gas und in der Gegenwart von einem Eisenoxid, $Fe_3O_4$, Katalysator und Dampf bei einer Temperatur im Bereich von 500 - 700°C und einem Druck von 0,1 - 10 Atmosphären umfaßt.

**2.** Verfahren nach Anspruch 1, worin der Eisenoxidkatalysator durch die Reduktion eines kommerziell verfügbaren $Fe_2O_3$-Katalysators erhalten wird.

**3.** Verfahren nach Anspruch 2, worin die Reduktion von $Fe_2O_3$ in situ vor dem Dehydrierungsschritt ausgeführt wird.

**4.** Verfahren nach einem der vorstehenden Ansprüche, worin der Reaktor vor der Einführung des Dehydrierungskatalysators in den Reaktor praktisch frei von Sauerstoff oder irgendwelchen oxidierenden Gasen gemacht wird.

**5.** Verfahren nach einem der vorstehenden Ansprüche, worin die Dehydrierungsreaktion, unter Verwendung einer Zufuhr, in der das relative molare Verhältnis von Dampf zu para-Cymol von 1,5 : 1 bis 20 : 1 ist, ausgeführt wird.

**6.** Verfahren nach einem der vorstehenden Ansprüche, worin die Dehydrierung bei einer Temperatur von 520 - 600°C ausgeführt wird.

**7.** Verfahren nach einem der vorstehenden Ansprüche, worin die Dehydrierung, unter Verwendung einer stündlichen Flüssigkeitsraum-Geschwindigkeit [liquid hourly space velocity] (LHSV) im Bereich von 0,1-1-10 $h^{-1}$, basierend auf dem para-Cymol in der Zufuhr, ausgeführt wird.

**8.** Verfahren nach einem der vorstehenden Ansprüche, worin deaktivierter Katalysator in dem Reaktor durch Erhitzen des deaktivierten Katalysators in der Gegenwart von Dampf regeneriert wird.

**9.** Verfahren nach Anspruch 8, worin der deaktivierte Katalysator in dem Dehydrierungsreaktor in situ durch Absperren der Kohlenwasserstoffzufuhr zu dem Reaktor und Erhitzen des deaktivierten Katalysators in der Gegenwart von Dampf regeneriert wird.

**10.** Verfahren nach einem der vorstehenden Ansprüche, worin das gebildete alpha,para-Dimethylstyrol aus den Reaktionsprodukten durch fraktionierte Destillation gewonnen wird.


**Revendications**

**1.** Procédé de production d'alpha, para-diméthylstyrène, ledit procédé comprenant la déshydrogénation de para-cymène dans un réacteur pratiquement en l'absence d'oxygène ou d'un gaz oxydant et en la présence d'un catalyseur à l'oxyde de fer, $Fe_3O_4$, et de vapeur d'eau à une température de 500 à 700°C et sous une pression de 0,1 à 10 atmosphères.

**2.** Procédé suivant la revendication 1, dans lequel le catalyseur à l'oxyde de fer est obtenu par réduction d'un catalyseur au $Fe_2O_3$, disponible dans le commerce.

3. Procédé suivant la revendication 2, dans lequel la réduction de $Fe_2O_3$ est effectuée in situ avant l'étape de déshydrogénation.

4. Procédé suivant l'une quelconque des revendications précédentes, dans lequel le réacteur est débarrassé pratiquement en totalité de l'oxygène ou de n'importe quels gaz oxydants avant introduction du catalyseur de déshydrogénation dans le réacteur.

5. Procédé suivant l'une quelconque des revendications précédentes, dans lequel la réaction de déshydrogénation est conduite en utilisant une charge dans laquelle le rapport molaire de la vapeur d'eau au para-cymène est compris dans l'intervalle de 1,5:1 à 20:1.

6. Procédé suivant l'une quelconque des revendications précédentes, dans lequel la déshydrogénation est effectuée à une température de 520 à 600°C.

7. Procédé suivant l'une quelconque des revendications précédentes, dans lequel la déshydrogénation est effectuée en utilisant une vitesse spatiale horaire de liquide (VSHL) de 0,1 à 10 $h^{-1}$, sur la base du para-cymène présent dans la charge.

8. Procédé suivant l'une quelconque des revendications précédentes, dans lequel tout catalyseur désactivé dans le réacteur est régénéré par chauffage du catalyseur désactivé en présence de vapeur d'eau.

9. Procédé suivant la revendication 8, dans lequel le catalyseur désactivé est régénéré in situ dans le réacteur de déshydrogénation par interruption de l'introduction de la charge d'hydrocarbure dans le réacteur et par chauffage du catalyseur désactivé en présence de vapeur d'eau.

10. Procédé suivant l'une quelconque des revendications précédentes, dans lequel l'alpha, para-diméthylstyrène formé est séparé des produits de réaction par distillation fractionnée.

# FIG.1

P-cymene and
water feed

Inert ceramic
beads

Reactor

Iron oxide catalyst
(Girdler G64C)

Product
cooler